(19) Europäisches Patentamt
European Patent Office
Office européen des brevets

(11) Veröffentlichungsnummer: **0 381 958 B1**

(12) **EUROPÄISCHE PATENTSCHRIFT**

(45) Veröffentlichungstag der Patentschrift: **23.08.95**

(51) Int. Cl.$^{6}$: **C07K 14/62**

(21) Anmeldenummer: **90100889.6**

(22) Anmeldetag: **17.01.90**

# (54) Verfahren zur Herstellung eines Insulinvorläufers.

(30) Priorität: **21.01.89 DE 3901719**

(43) Veröffentlichungstag der Anmeldung:
**16.08.90 Patentblatt 90/33**

(45) Bekanntmachung des Hinweises auf die Patenterteilung:
**23.08.95 Patentblatt 95/34**

(84) Benannte Vertragsstaaten:
**AT BE CH DE DK ES FR GB GR IT LI LU NL SE**

(56) Entgegenhaltungen:
**EP-A- 0 037 255**

(73) Patentinhaber: **HOECHST AKTIENGESELLSCHAFT**

**D-65926 Frankfurt am Main (DE)**

(72) Erfinder: **Dörschug, Michael, Dr.**
**Sonnenleite 20**
**D-4630 Bochum 7 (DE)**

**EP 0 381 958 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Insulin ist ein Molekül, das aus 2 Polypeptidketten besteht, die über Disulfidbrücken miteinander verknüpft sind. Die A-Kette besteht aus 21 Aminosäuren, die B-Kette aus 30 Aminosäuren. Diese beiden Ketten sind im Vorläufermoelkül, dem Proinsulin, durch ein Peptid, das C-Peptid, miteinander verknüpft. Das C-Peptid im Humanproinsulin besteht aus 35 Aminosäuren. Das C-Peptid wird im Rahmen des Reifungsprozesses des Hormons durch spezifische Proteasen abgespalten und das Proinsulin so in Insulin überführt (Davidson et al., Nature 333, 93-96, 1988). Neben den natürlich vorkommenden C-Peptiden sind in der Literatur eine Vielzahl von Verbindungsmöglichkeiten zwischen A-Kette und B-Kette beschrieben (Yanaihara et al., Diabetes 27, 149-160 (1978), Busse et al., Biochemistry 15, 1649-1657 (1971), Geiger et al., Biochem. Biophys. Res. Com. 55, 60-66 (1973)).

Im Rahmen der Gentechnologie ist es heute möglich, Insulin aus gentechnisch veränderten Mikroorganismen herzustellen. Wird als Mikroorganismus E. coli verwendet, so wird das Produkt häufig als Fusionsprotein exprimiert, d.h. das Produkt wird mit einem bakterieneigenen Protein gekoppelt, z.B. mit der β-Galaktosidase. Dieses Fusionsprotein fällt in der Zelle aus und ist so vor proteolytischem Abbau geschützt. Nach Zellaufschluß wird der Fusionsproteinanteil chemisch oder enzymatisch abgespalten und die 6 Cysteine des Insulinvorläufers mittels oxidativer Sulfitolyse in ihre S-Sulfonate ($-S-SO_3^-$) überführt. Aus diesem sogenannten Präproinsulin-S-Sulfonat muß in einem folgenden Schritt natives Präproinsulin unter Ausbildung der 3 korrekten Disulfidbrücken erzeugt werden.

Dieser Schritt erfolgt z.B. nach dem in der EP-B-00 37 255 beschriebenen Verfahren durch Umsetzung des Ausgangs-S-Sulfonats mit einem Mercaptan in einer Menge, die 1 bis 5 SH-Reste pro $SSO_3^-$ -Rest ergibt, in wäßrigem Medium bei einem pH-Wert von 7 bis 11,5 und einer S-Sulfonatkonzentration von bis zu 10 mg pro ml wäßriges Medium vorzugsweise in Abwesenheit eines Oxidationsmittels.

Für den Erhalt hoher Faltungsausbeuten - d.h. hoher Ausbeuten an Präproinsulin mit den "richtigen" Peptidsequenz-Verknüpfungen (-S-S-Brücken von A6 nach A11, von A7 nach B7 und von A20 nach B19) - ist hierbei jedoch die Einhaltung des angegebenen - eng begrenzten - $SH/SSO_3^-$-Verhältnisses notwendig, was eine nicht unerhebliche Sorgfalt bei der Verfahrensdurchführung und insbesondere eine - nicht ganz einfache - quantitative Bestimmung der $SSO_3^-$-Gruppen in dem Ausgangs-S-Sulfonat erfordert.

Überraschenderweise wurde nun gefunden, daß man zu - von dem $SH/SSO_3^-$-Verhältnis innerhalb weiter Grenzen praktisch unabhängigen - hohen Faltungsausbeuten kommt, wenn man bei einem höheren - d.h. oberhalb von 5:1 liegenden - $SH/SSO_3^-$-Verhältnis in Gegenwart eines organischen Redox-Systems oder von Verbindungen, welche unter den Reaktionsbedingungen ein solches organisches Redox-System bilden, arbeitet; anstelle der $SSO_3^-$-Gruppen können in dem entsprechenden Ausgangsprodukt auch andere S-Schutzgruppen vorhanden sein; außerdem können die Peptidsequenzen der Insulin-A- und -B-Kette auch durch den Austausch einer oder mehrerer Aminosäuren verändert sein.

Das höhere $SH/SSO_3^-$-(bzw. S-Schutzgruppen)-Verhältnis bedeutet, daß man - sofern man nur eine bestimmte Mercaptan-Mindestmenge überschreitet - praktisch unabhängig von der Höhe des Mercaptan-Überschusses ohne wesentliche Ausbeute-Beeinträchtigung arbeiten kann; dadurch erübrigt sich auch eine exakte quantitative Bestimmung der $SSO_3^-$-(bzw. S-Schutz-)Gruppen-Bestimmung in dem entsprechenden Insulin-Ausgangsprodukt, was einen erheblichen Verfahrensvorteil darstellt.

Das Arbeiten praktisch unabhängig von der Höhe des Mercaptan-Überschusses wird durch die Gegenwart eines organischen Redox-System bzw. von Verbindungen, welche unter den Reaktionsbedingungen ein solches organisches Redox-System bilden, ermöglicht.

Es ist zwar bekannt, daß reduziertes Proinsulin - d.i. Proinsulin, dessen S-S-Brücken z.B. mit einem Mercaptan reduktiv zu SH-Gruppen gespalten wurden - zu dem ursprünglichen Proinsulin reoxidiert werden kann und daß diese Reoxidation durch die Gegenwart von Dehydroascorbinsäure beschleunigt wird - vgl. D.F. Steiner und J.L. Clark, Proc. Natl. Mod. Sci. USA 60, 622-624 (1968); selbst wenn sich unter den dort beschriebenen Reaktionsbedinungen aus Dehydroascorbinsäure ein Redox-System aus Dehydroascorbinsäure und Ascorbinsaure bilden sollte, geht es dort um die Oxidation von reduziertem Proinsulin mit ungeschützten S-Gruppen, während im vorliegenden erfindungsgemäßen Fall als Ausgangsprodukte nur Insulin-Vorprodukte mit geschützten S-Gruppen in Frage kommen. Außerdem enthält die genannte Literaturstelle von D.F. Steiner und J.L. Clark a.a.O. keinerlei Hinweis oder Anregung in Richtung auf den Einsatz und die Wirkung eines organischen Redox-Systems bei der Rekombination von Insulin-Vorläufer-Produkten, welche S-Schutzgruppen enthalten, mittels Mercaptanen.

Erfindungsgegenstand ist nun im einzelnen ein Verfahren zur Herstellung eines Insulin-Vorläufers der Formel I

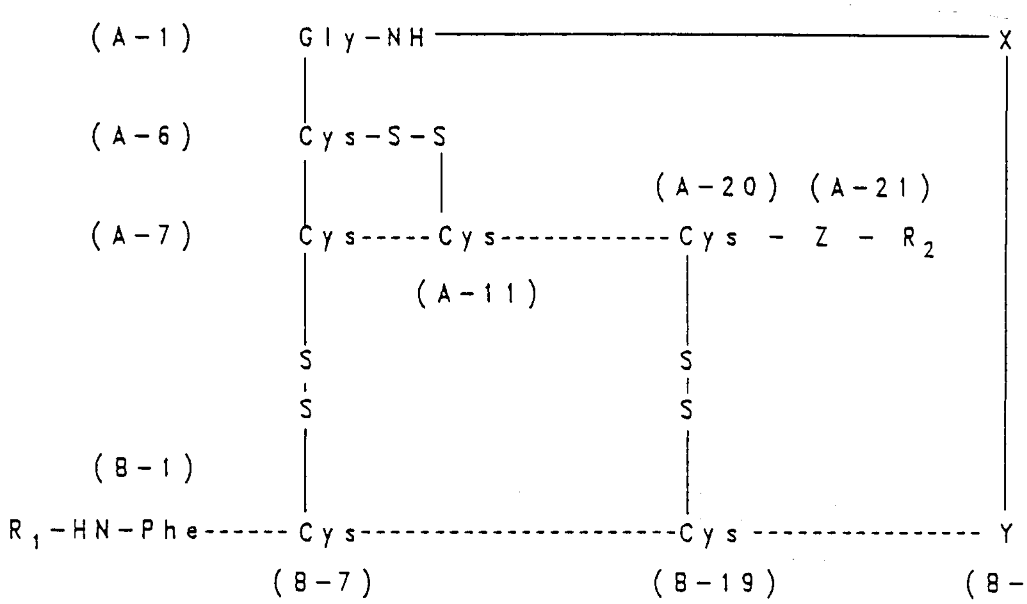

(I)

worin

| | |
|---|---|
| $R_1$ | = H, ein chemisch oder enzymatisch abspaltbarer Aminosäure- oder Peptidrest, |
| $R_2$ | = OH oder ein Aminosäure- oder Peptidrest, vorzugsweise OH |
| X | = ein die Insulin A- und B-Kette verbindender Rest, vorzugsweise ein Aminosäure- oder Peptidrest. |
| Y | = Rest einer genetisch kodierbaren Aminosäure, vorzugsweise Thr, Ala, Ser, insbesondere Thr, |
| Z | = Rest einer genetisch kodierbaren Aminosäure, vorzugsweise Asn, Gln, Asp, Glu, Gly, Ser, Thr, Ala oder Met, insbesondere Asn, |
| A1-A20 und B1-B29 | = unmutierte oder durch Austausch einer oder mehrerer Aminosäuren mutierte Peptidsequenzen des Insulins, vorzugsweise die unmutierten Peptidsequenzen des Human-, Schweine- oder Rinderinsulins, insbesondere des Human- oder Schweineinsulins, |

durch Umsetzung eines Vorproduktes mit geschützten Cys-S-Gruppen und einem Mercaptan in wäßrigem Medium, dadurch gekennzeichnet, daß man ein Vorprodukt mit geschützten Cys-S-Gruppen der Formel II

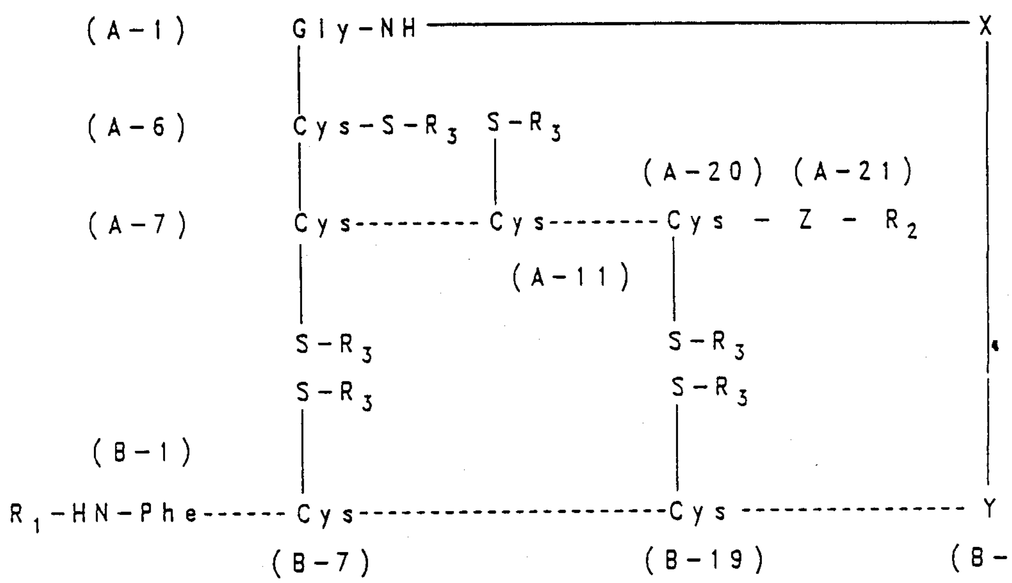

(II)

worin $R_1$, $R_2$, X, Y, Z, A1-A20 und B1-B29 die gleiche Bedeutung wie in Formel I besitzen und

$R_3$ = eine Cys-S-Schutzgruppe, vorzugsweise die $-SO_3$-Gruppe,

mit einem Mercaptan in einer Menge entsprechend einem (Mercaptan)-SH/ Cys-S-$R_3$ (in Formel II)-Verhältnis von 10 bis 30, einem pH-Wert zwischen 9,5 und 11, in Gegenwart eines organischen Redox-Systems oder mindestens einer organischen Verbindung, welcher unter den Reaktionsbedingungen ein solches organisches Redox-System bildet, ausgewählt aus der Gruppe Ascorbinsäure, Dehydroascorbinsäure, Brenzkatechin, o-Chinon, Hydrochinon und p-Chinon, wobei man das Mercaptan und die das organische Redox-System bildende(n) Verbindung(en) in einem Verhältnis von 1 Grammäquivalent Mercaptan zu 1/10 bis 10 Mol das organische Redox-System bildende(n) Verbindung(en) einsetzt, umsetzt.

Wenn in den Formeln I und II $R_1$ = H, handelt es sich um Proinsulin bzw. sich vom Proinsulin ableitende Produkte;

Wenn $R_1$ = chemisch oder enzymatisch abspaltbarer Aminosäure- oder Peptidrest, handelt es sich um Präproinsulin sowie sich davon ableitende Produkte.

Chemisch abspaltbare Aminosäurereste sind solche, welche z. B. mittels BrCN oder N-Bromsuccinimid abgespalten werden können; dies sind z. B. Methionin (Met) oder Tryptophan (Trp).

Enzymatisch abspaltbare Aminosäurereste sind solche, welche z.B. mittels Trypsin abspaltbar sind (wie z.B. Arg oder Lys).

Chemisch oder enzymatisch abspaltbare Peptidreste sind Peptidreste mit wenigstens 2 Aminosäuren.

Sämtliche für $R_1$ in Frage kommenden Aminosäuren sind bevorzugt aus der Gruppe der natürlichen Aminosäuren, d.s. hauptsächlich Gly, Ala, Ser, Thr, Val, Leu, Ile, Asn, Gln, Cys, Met, Tyr, Phe, Pro, Hyp, Arg, Lys, Hyl, Orn, Cit und His.

$R_2$ ist OH oder - ähnlich $R_1$ - ebenfalls ein Aminosäure-oder Peptidrest, wobei die Bedeutung OH bevorzugt ist. Die Aminosäuren (auch diejenigen, welche den - aus mindestens 2 Aminosäureresten bestehenden - Peptidrest bilden) stammen - wie bei $R_1$ - vorzugsweise aus der Gruppe der natürlichen Aminosäuren.

X ist ein die Insulin-A- und -B-Kette verbindender Rest, vorzugsweise ein Aminosäure- oder Peptidrest.

Wenn X ein Aminosäurerest ist, ist der Rest von Arg oder Lys bevorzugt; wenn X ein Peptidrest ist, ist der Rest eines natürlich vorkommenden C-Peptids - insbesondere des Human-, Schweine- oder Rinder-Insulin-C-Peptids - bevorzugt.

Genetisch kodierbare Aminosäuren - für Y - sind (jeweils in der L-Form): Gly, Ala, Ser, Thr, Val, Leu, Ile, Asp, Asn, Glu, Gln, Cys, Met, Arg, Lys, His, Tyr, Phe, Trp, Pro.

Bevorzugte genetisch kodierbare Aminosäuren sind Thr, Ala und Ser, insbesondere Thr.

Z kann - wie Y ebenfalls den Rest einer genetisch kodierbaren Aminosäure bedeuten, wobei hier aber Asn, Gln, Asp, Glu, Gly, Ser, Thr, Ala und Met, insbesondere Asn, bevorzugt sind.

A1 - A20 und B1 - B29 können im Prinzip die unmutierten oder durch Austausch einer oder mehrerer Aminosäuren mutierten Peptidsequenzen aller möglichen Insuline sein; die Mutanten können nach bekannten gentechnologischen Verfahren (site directed mutagenesis) erzeugt werden. Bevorzugt sind jedoch die unmutierten Peptidsequenzen des Human-, Schweine- oder Rinderinsulins, insbesondere des Human- oder Schweineinsulins (die A1 - A20 und B1 - B29-Sequenzen des Human- und Schweineinsulins sind identisch).

Der nur in Formel II vorkommende Rest $R_3$ bedeutet eine praktisch beliebige Cys-S-Schutzgruppe, vorzugsweise jedoch die $-SO_3$-Gruppe.

Das Ausgangsprodukt der Formel II kann im Prinzip in einem weiten Konzentrationsbereich - zweckmäßig zwischen etwa 10 $\mu$g und 10 mg/ml Lösung - eingesetzt werden, wobei bekanntermaßen geringere Konzentrationen zu höheren Renaturierungsausbeuten führen, da bei geringen Proteinkonzentrationen die Aggregationsneigung vermindert ist. Bevorzugte Konzentrationen liegen zwischen etwa 0,1 mg und 0,5 mg/ml.

Als Mercaptane kommen für die erfindungsgemäße Umsetzung im Prinzip alle möglichen organischen Verbindungen mit SH-Gruppen in Betracht; bevorzugt sind Mercaptoethanol, Tioglykolsäure, Glutathion und Cystein, insbesondere Mercaptoethanol und Cystein. Die Mercaptane können einzeln oder in Mischung eingesetzt werden.

Die Mercaptan-Menge ist so (zu) bemessen, daß das Verhältnis von deren SH-Gruppen zu den Cys-$SR_3$-Gruppen des Ausgangsmaterial der Formel II von 10 bis 30 liegt.

Bevorzugte organische Redox-Systeme sind die aus den Verbindungspaaren

Ascorbinsäure + Dehydroascorbinsäure,

Brenzkatechin + o-Chinon und

Hydrochinon + p-Chinon

bestehende Kombinationen,

und bevorzugte Einzelverbindungen, welche unter den Reaktionsbedingungen ein solches Redox-System

bilden, sind die Einzelkomponenten dieser Verbindungspaare.

Bei der erfindungsgemäßen Umsetzung können nun die jeweiligen organischen Redox-Systeme, bestehend z. B. aus den Verbindungspaaren Ascorbinsäure + Dehydroascorbinsäure, Brenzkatechin + o-Chinon, Hydrochinon + p-Chinon, Naphthohydrochinon + Naphthochinon, etc. in praktisch beliebigem Verhältnis (vorzugsweise in etwa äquimolarem Verhältnis) eingesetzt werden. Es ist aber auch möglich, nur die jeweiligen Einzelkomponenten dieser Verbindungspaare - also z. B. nur Ascorbinsäure oder nur Dehydroascorbinsäure oder nur Hydrochinon usw. - zuzusetzen, weil sich in dem Reaktionsmedium die zu dem Redox-Verbindungspaar gehörige, jeweils andere Komponente (Dehydroascorbinsäure bzw. Ascorbinsäure bzw. p-Chinon usw.) bildet.

Ganz besonders bevorzugt sind Ascorbinsäure und/oder Dehydroascorbinsäure.

Die eingesetzte Menge der das organische Redox-System bildende(n) Verbindung(en) kann in weiten Gruppen variiert werden. Bezogen auf ein Grammäquivalent Mercaptan (= Molekulargewicht des eingesetzten Mercaptans in g/Zahl der SH-Gruppen im Mercaptan-Molekül), kann die Molzahl der das organische Redox-System bildende(n) Verbindung(en) zwischen etwa 1/10 und 10, gewählt werden.

Die erfindungsgemäße Umsetzung wird im alkalischen pH-Bereich zwischen etwa 9,5 und 11 durchgeführt. Zur Aufrechterhaltung des gewünschten pH-Wertes ist der Zusatz einer Puffersubstanz zweckmäßig, wobei Art und Ionenstärke des Puffers einen gewissen Einfluß auf die Faltungsausbeute haben. Es ist vorteilhaft, die Ionenstärke gering zu halten, wobei ein Bereich von etwa 1 mM (mM = millimolar) bis 1 M (M = molar), insbesondere ein solcher von etwa 5 mM bis 50 mM, bevorzugt ist. Als Puffersubstanzen können z. B. Boratpuffer, Carbonatpuffer oder Glycinpuffer verwendet werden, wobei letzterer bevorzugt ist.

Als allgemeiner Bereich der Reaktionstemperatur kann ein solcher zwischen 0 und 45 °C angegeben werden; bevorzugt ist ein Bereich von etwa 4 bis 8 °C.

Die Überschichtung der Renaturierungslösung mit bestimmten Gasen, wie z. B. Sauerstoff, Stickstoff oder Helium hat keinen merklichen Einfluß auf die Renaturierungsausbeute.

Die Umsetzungsdauer liegt im allgemeinen zwischen etwa 2 und 24 Stunden, bevorzugt zwischen etwa 6 und 16 Stunden.

Nach beendeter Umsetzung (was z.B. durch HPLC festgestellt werden kann) wird in bekannter Weise aufgearbeitet, wie z.B. auch in der eingangs erwähnten EP-B-00 37 255 beschrieben.

Das "richtig" gefaltete Produkt der Formel I kann dann enzymatisch oder chemisch nach bekannten Techniken in das entsprechende Insulin überführt werden.

Die folgenden Beispiele sollen die Erfindung nun weiter erläutern und auch die Vorteile gegenüber dem Stand der Technik verdeutlichen.

Als Ausgangsmaterial für die Renaturierungsexperimente wurde Präproinsulin-S-$SO_3^-$ verwendet, das auf gentechnologische Weise gewonnen wurde.

Als Expressionssystem wurde E. coli verwendet. In E. coli ist das Gen für Proinsulin mit einem Teil des $\beta$-Galaktosidasegens gekoppelt und wird als Fusionsprotein synthetisiert, das in der Zelle ausfällt und in den Polkappen abgelagert wird (nach dem Verfahren der DE-A-38 05 150). Nach Zellaufschluß wird der Fusionsproteinanteil mittels Halogencyan abgespalten (gemäß dem Verfahren der DE-A-34 40 988) und anschließend der oxidativen Sulfitolyse unterworfen (R.C. Marshall und A.S. Ingles in A. Darbre (Hrsg.) "Practical Protein Chemistry - A Handbook" (1986), S. 49 - 53), um die 6 Cysteine in ihre S-Sulfonatform zu überführen. Das so hergestellte Präproinsulin-S-$SO_3^-$ (der Präfix "Prä" bedeutet, daß das Proinsulin an seinem N-Terminus um 5 Aminosäuren verlängert ist) wird anschließend mittels Ionenaustauscher in an sich bekannter Weise angereichert, gefällt und gefriergetrocknet. Das so gewonnene gefriergetrocknete Ausgangsmaterial hat laut HPLC-Bestimmung einen Gehalt von 60 %.

1. **Abhängigkeit der Faltungsausbeute vom Mercaptan/Ascorbinsäure-Verhältnis**

Präproinsulin-S-$SO_3$ (60 %) wird mit einer Konzentration von 0,33 mg/ml in 20 mM Glycinpuffer, pH 10,5, gelöst, was einer Präproinsulin-S-$SO_3^-$ Konzentration von 0,2 mg/ml entspricht. Es werden pro Ansatz 20 ml eingesetzt, zu denen 480 µl einer 0,1 M Cysteinlösung (~20facher molarer Überschuß pro S-$SO_3^-$-Gruppe) und zwischen 0 und 480 µl einer 0,1 M Ascorbinsäurelösung gegeben werden. Die Renaturierungstemperatur beträgt 8 °C, die Reaktionsdauer 16 Stunden.

| Ascorbinsäure (0,1 M) | Faltungsausbeute | |
|---|---|---|
| 0 | 25 % | Vergleich |
| 24 µl | 28 % | erfindungsgemäß |
| 48 µl | 53 % | erfindungsgemäß |
| 96 µl | 78 % | erfindungsgemäß |
| 480 µl | 81 % | erfindungsgemäß |

Dieses Beispiel zeigt den Einfluß der Redoxverbindung auf die Faltungsausbeute. Während es bei dem Ansatz ohne Ascorbinsäure überwiegend zur Bildung von fehlgefalteten Proteinen kommt, steigt die Faltungsausbeute in Gegenwart der Redoxverbindung an.

2. **Abhängigkeit der Faltungsausbeute vom Mercaptan/Ascorbinsäureüberschuß**

Die Einwaage, Volumen, Puffer, Reaktionszeit und -temperatur und pH-Wert entsprechen dem Versuch 1. Es werden jeweils äquimolare Mengen an Cystein und Ascorbinsäure zugegeben, wobei der molare Überschuß pro S-$SO_3^-$-Gruppe zwischen 2,5 und 100 liegt.

| zugegebene Menge an Cystein und Ascorbinsäure | Überschuß pro S-$SO_3^-$-Gruppe | Faltungsausbeute | |
|---|---|---|---|
| 60 µl | 2,5 | 38 % | Vergleich |
| 120 µl | 5 | 70 % | Vergleich |
| 240 µl | 10 | 83 % | erfindungsgemäß |
| 480 µl | 20 | 85 % | erfindungsgemäß |
| 1200 µl | 50 | 81 % | erfindungsgemäß |
| 2400 µl | 100 | 72 % | erfindungsgemäß |

Dieses Beispiel zeigt, daß - sobald man eine bestimmte Mercaptanmindestmenge überschreitet - die Faltungsausbeute in einem weiten Bereich weitgehend unabhängig vom Mercaptanüberschuß ist.

3. **Abhängigkeit der Faltungsausbeute vom pH-Wert**

Die Einwaage, Volumen, Pufferkonzentration, Reaktionszeit und -temperatur entsprechen Versuch 1. Bei dem Versuch werden 480 µl 0,1 M Cysteinlösung und 480 µl 0,1 M Ascorbinsäurelösung zugegeben (~20facher molarer Überschuß pro S-$SO_3^-$-Gruppe)

| pH-Wert | Faltungsausbeute |
|---|---|
| 11,0 | 79 % |
| 10,5 | 81 % |
| 10,0 | 66 % |
| 9,5 | 53 % |
| 9,0 | 46 % |
| 8,5 | 33 % |
| 8,0 | 24 % |

4. **Abhängigkeit der Faltungsausbeute vom Mercaptantyp**

Die Einwaage, Volumen, Pufferkonzentration, Reaktionszeit und -temperatur und pH-Wert entsprechen Versuch 1. Bei dem Versuch werden jeweils 480 µl der entsprechenden 0,1 M Mercaptanlösung und 400 µl 0,1 M Ascorbinsäurelösung zugegeben (~20facher molarer Überschuß pro $S\text{-}SO_3^-$-Gruppe)

| Mercaptan | Faltungsausbeute |
|---|---|
| Cystein | 81 % |
| Mercaptoethanol | 86 % |
| Glutathion | 75 % |
| Thioglykolsäure | 74 % |
| 3-Mercapto-1,2-propandiol | 76 % |

5. **Abhängigkeit der Faltungsausbeute von der Puffersubstanz und von der Ionenstärke des Puffers**

Die Einwaage, Volumen, Reaktionszeit und -temperatur und pH-Wert entsprechen Versuch 1. Bei dem Versuch werden jeweils 480 µl der entsprechenden 0,1 M Mercaptanlösung und 480 µl 0,1 M Ascorbinsäurelösung zugegeben (~20facher molarer Überschuß pro $S\text{-}SO_3^-$-Gruppe).

| Puffer | Faltungsausbeute |
|---|---|
| 10 mM Glycin | 89 % |
| 100 mM Glycin | 82 % |
| 10 mM Borat | 82 % |
| 100 mM Borat | 51 % |
| 10 mM Carbonat | 88 % |
| 100 mM Carbonat | 72 % |

6. **Abhängigkeit der Faltungsausbeute von der Reaktionszeit**

Die Einwaage, Volumen, Pufferkonzentration, pH-Wert und Reaktionstemperatur entsprechen dem Versuch 1. Es werden jeweils 480 µl einer 0,1 M Mercaptoethanollösung und 480 µl einer 0,1 M Ascorbinsäurelösung zugegeben (~20facher molarer Überschuß pro $S\text{-}SO_3^-$-Gruppe)

| Reaktionszeit (h) | Faltungsausbeute |
|---|---|
| 0,25 | 25 % |
| 0,5 | 40 % |
| 1 | 46 % |
| 2 | 61 % |
| 4 | 70 % |
| 6 | 77 % |
| 8 | 83 % |
| 24 | 79 % |

7. **Abhängigkeit der Faltungsausbeute von der Präproinsulin-S-Sulfonat-Konzentration**

Volumen, Pufferzusammensetzung, pH-Wert, Reaktionszeit und Temperatur entsprechen Versuch 1. Zu der eingesetzten Präproinsulin-S-Sulfonatmenge werden jeweils soviel aus einer Mercaptoethanolstammlösung und Ascorbinsäurestammlösung zugegeben, daß ein ~20facher molarer Überschuß pro $S\text{-}SO_3^-$-Gruppe besteht.

| Präproinsulin-S-$SO_3^-$-Konzentration | Faltungsausbeute |
|---|---|
| 0,1 ml/ml | 86 % |
| 0,2 ml/ml | 84 % |
| 0,3 ml/ml | 78 % |
| 0,4 ml/ml | 65 % |
| 0,5 ml/ml | 55 % |
| 1,0 ml/ml | 19 % |
| 2,5 ml/ml | 5 % |

8. **Abhängigkeit der Faltungsausbeute vom Ascorbinsäure/Mercaptoethanolüberschuß bei modifiziertem Ausgangsmaterial**

Zum Einsatz kommt ein Vorläufermolekül, bei dem A- und B-Kette des Insulins nur durch ein Arginin verknüpft sind. Die gentechnologische Gewinnung dieses Moleküls erfolgt wie am Anfang der Versuchsbeschreibungen beschrieben. Das gefriergetrocknete Material hat einen Gehalt von 60 % und wird mit einer Konzentration von 0,5 mg/ml in 20 mM Glycinpuffer, pH 10,5, gelöst, was einer Vorläuferkonzentration von 0,3 mg/ml entspricht. Reaktionszeit und -temperatur entsprechen Versuch 1, der molare Überschuß des Ascorbinsäure/Mercaptangemisches variiert zwischen 2,5 und 50fach.

| Überschuß pro S-$SO_3^-$-Gruppe | Faltungsausbeute | |
|---|---|---|
| 2,5 | 56 % | Vergleich |
| 5 | 61 % | |
| 10 | 74 % | erfindungsgemäß |
| 20 | 75 % | |
| 50 | 55 % | |

9. **Abhängigkeit der Faltungsausbeute vom Redox-System**

Einwaage, Volumen, Pufferzusammensetzung, pH-Wert, Reaktionszeit und -temperatur entsprechen Versuch 1. Es wird ein ~20facher molarer Überschuß an Mercaptoethanol/S-$SO_3^-$-Gruppe und ein ~20facher molarer Überschuß des entsprechenden Redox-Partners hinzugegeben.

| Redox-Partner | Faltungsausbeute |
|---|---|
| Ascorbinsäure | 77 % |
| Dehydroascorbinsäure | 70 % |
| Brenzkatechin | 66 % |
| Hydrochinon | 40 % |
| Benzo(-p-)chinon | 23 % |

## Patentansprüche

**1.** Verfahren zur Herstellung eines Insulinvorläufers der Formel I

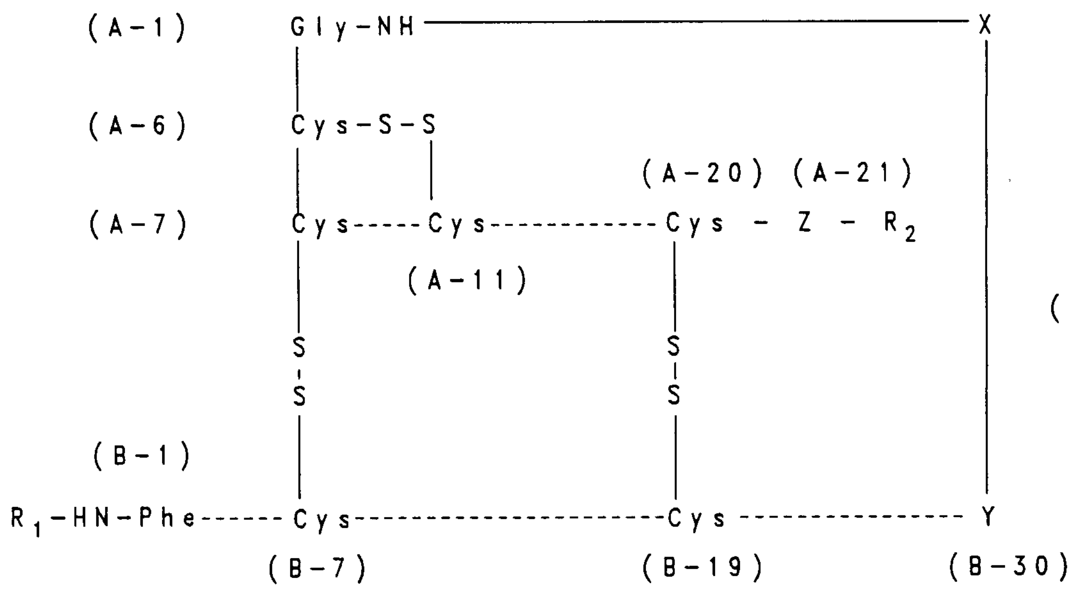

(I)

worin

| | |
|---|---|
| $R_1$ | = H, ein chemisch oder enzymatisch abspaltbarer Aminosäure- oder Peptidrest, |
| $R_2$ | = OH oder ein Aminosäure- oder Peptidrest, vorzugsweise OH |
| X | = ein die Insulin A- und B-Kette verbindender Rest, vorzugsweise ein Aminosäure- oder Peptidrest. |
| Y | = Rest einer genetisch kodierbaren Aminosäure, vorzugsweise Thr, Ala, Ser, insbesondere Thr, |
| Z | = Rest einer genetisch kodierbaren Aminosäure, vorzugsweise Asn, Gln, Asp, Glu, Gly, Ser, Thr, Ala oder Met, insbesondere Asn, |
| A1-A20 und B1-B29 | = unmutierte oder durch Austausch einer oder mehrerer Aminosäuren mutierte Peptidsequenzen des Insulins, vorzugsweise die unmutierten Peptidsequenzen des Human-, Schweine- oder Rinderinsulins, insbesondere des Human- oder Schweineinsulins, |

durch Umsetzung eines Vorproduktes mit geschützten Cys-S-Gruppen und einem Mercaptan in wäßrigem Medium, dadurch gekennzeichnet, daß man ein Vorprodukt mit geschützten Cys-S-Gruppen der Formel II

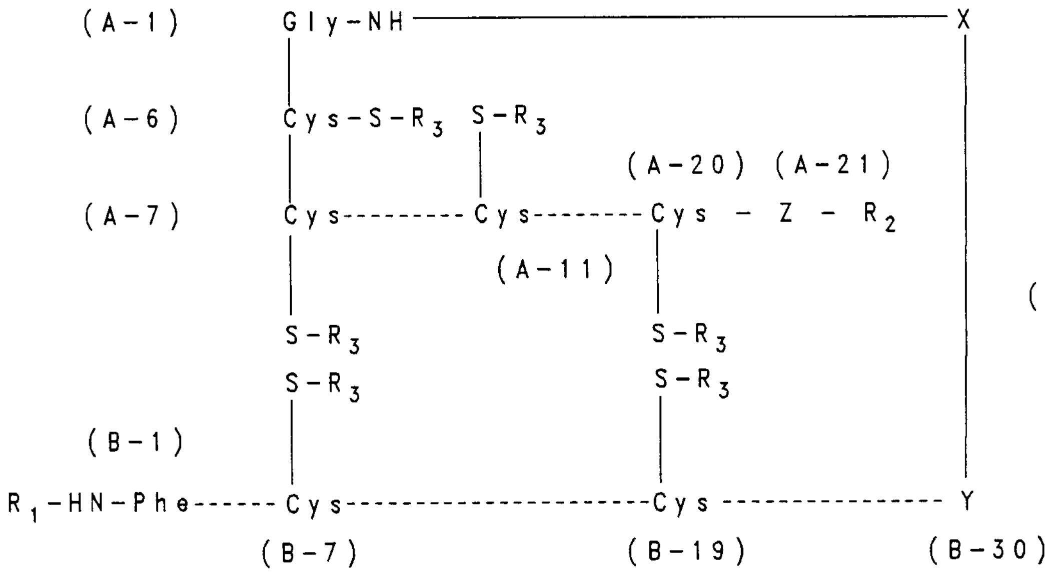

(II)

worin $R_1$, $R_2$, X, Y, Z, A1-A20 und B1-B29 die gleiche Bedeutung wie in Formel I besitzen und

$R_3$ = eine Cys-S-Schutzgruppe, vorzugsweise die -$SO_3^-$-Gruppe,

mit einem Mercaptan in einer Menge entsprechend einem (Mercaptan-)SH/Cys-S-$R_3$ (in Formel II)-Verhältnis von 10 bis 30, einem pH-Wert zwischen 9,5 und 11, in Gegenwart eines organischen Redox-Systems oder mindestens einer organischen Verbindung, welcher unter den Reaktionsbedingungen ein solches organisches Redox-System bildet, ausgewählt aus der Gruppe Ascorbinsäure, Dehydroascorbinsäure, Brenzkatechin, o-Chinon, Hydrochinon und p-Chinon, wobei man das Mercaptan und die das organische Redox-System bildende(n) Verbindung(en) in einem Verhältnis von 1 Grammäquivalent Mercaptan zu 1/10 bis 10 Mol das organische Redox-System bildende(n) Verbindung(en) einsetzt, umsetzt.

**2.** Verfahren nach Anspruch 1, dadurch gekennzeichnet, daß man als Mercaptan Mercaptoethanol und/oder Cystein verwendet.

**3.** Verfahren nach einem oder mehreren der Ansprüche 1 bis 2, dadurch gekennzeichnet, daß man in Gegenwart von Ascorbinsäure und/oder Dehydroascorbinsäure arbeitet.

## Claims

**1.** A process for the preparation of an insulin precursor of the formula I

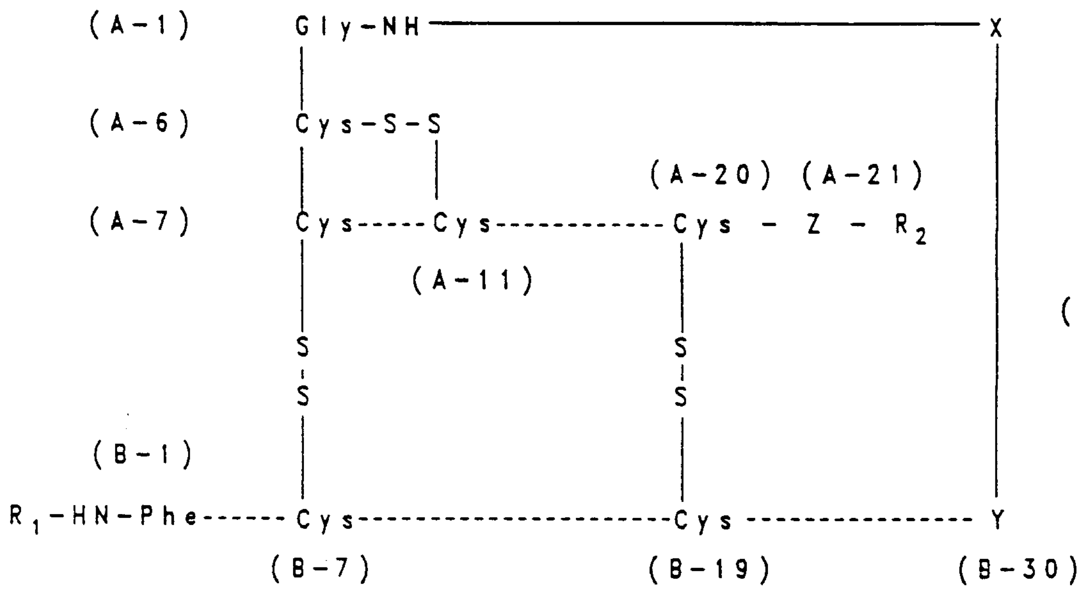

(I)

in which

| | |
|---|---|
| $R_1$ = | H or an amino acid or peptide radical which can be split off chemically or enzymatically, |
| $R_2$ = | OH or an amino acid or peptide radical, preferably OH, |
| X = | a radical which bonds the insulin A and B chain, preferably an amino acid or peptide radical, |
| Y = | the radical of a genetically encodable amino acid, preferably Thr, Ala or Ser, in particular Thr, |
| Z = | the radical of a genetically encodable amino acid, preferably Asn, Gln, Asp, Glu, Gly, Ser, Thr, Ala or Met, in particular Asn, and |
| A1-A20 and B1-B29 | = peptide sequences of insulin which are non-mutated or mutated by replacement of one or more amino acids, preferably non-mutated peptide sequences of human, porcine or bovine insulin, in particular human or porcine insulin, |

by reaction of a precursor having protected Cys-S groups and a mercaptan in aqueous medium, which comprises reacting a precursor having protected Cys-S groups, of the formula II

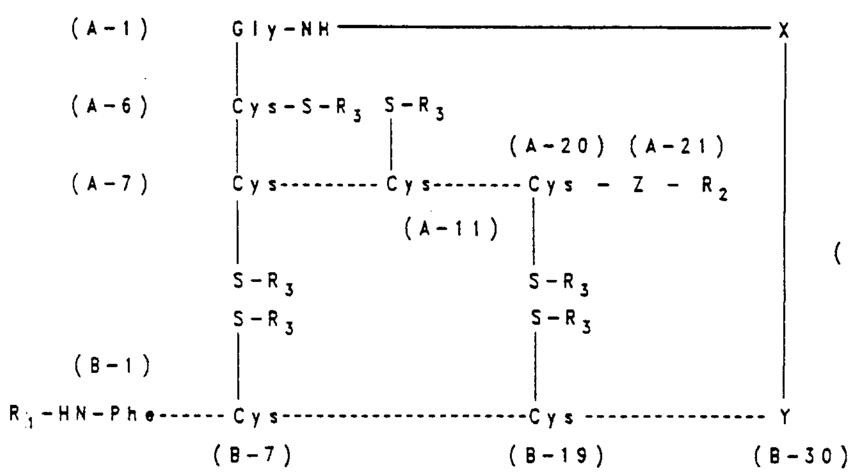

(II)

in which $R_1$, $R_2$, X, Y, Z, A1-A20 and B1-B29 have the same meaning as in formula I and

$R_3$ = a Cys-S protective group, preferably the $-SO_3^-$ group,

with a mercaptan in an amount corresponding to a (mercaptan) SH/Cys-S-$R_3$ (in formula II) ratio of 10 to 30 at a pH of between 9.5 and 11, in the presence of an organic redox system or at least one organic compound which forms such an organic redox system under the reaction conditions, chosen from the group consisting of ascorbic acid, dehydroascorbic acid, pyrocatechol, o-quinone, hydroquinone and p-quinone, the mercaptan and the compound(s) which form(s) the organic rexox system being employed in a ratio of 1 gram equivalent of mercaptan to 1/10 to 10 mol of the compound(s) forming the organic redox system.

**2.** The process as claimed in claim 1, wherein mercaptoethanol and/or cysteine are used as the mercaptan.

**3.** The process as claimed in one or more of claims 1 to 2, carried out in the presence of ascorbic acid and/or dehydroascorbic acid.

**Revendications**

**1.** Procédé pour la préparation d'un précurseur d'insuline de formule I

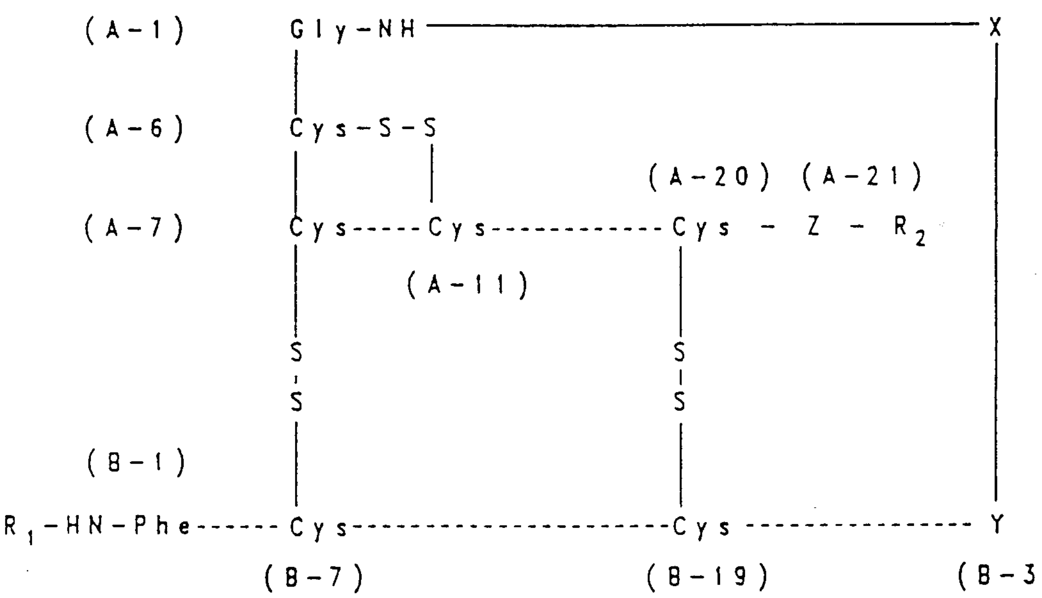

(I)

dans laquelle

| | |
|---|---|
| $R_1$ | = H, un reste d'aminoacide ou de peptide séparable par voie chimique ou enzymatique, |
| $R_2$ | = OH ou un reste d'aminoacide ou de peptide, de préférence OH, |
| X | = un radical reliant la chaîne A et la chaîne B de l'insuline, de préférence un reste d'aminoacide ou de peptide, |
| Y | = le reste d'un aminoacide génétiquement codable, de préférence Thr, Ala, Ser, en particulier Thr, |
| Z | = le reste d'un aminoacide génétiquement codable, de préférence Asn, Gln, Asp, Glu, Gly, Ser, Thr, Ala ou Met, en particulier Asn, |
| A1-A20 et B1-B29 | = séquences peptidiques de l'insuline non mutées ou mutées par remplacement d'un ou plusieurs aminoacides, de préférence les séquences peptidiques non mutées de l'insuline humaine, porcine ou bovine, en particulier de l'insuline humaine ou porcine, |

par la réaction d'un précurseur à atomes S de Cys protégés, et d'un mercaptan en milieu aqueux, caractérisé en ce que l'on fait réagir un précurseur à atomes S de Cys protégés, de formule II

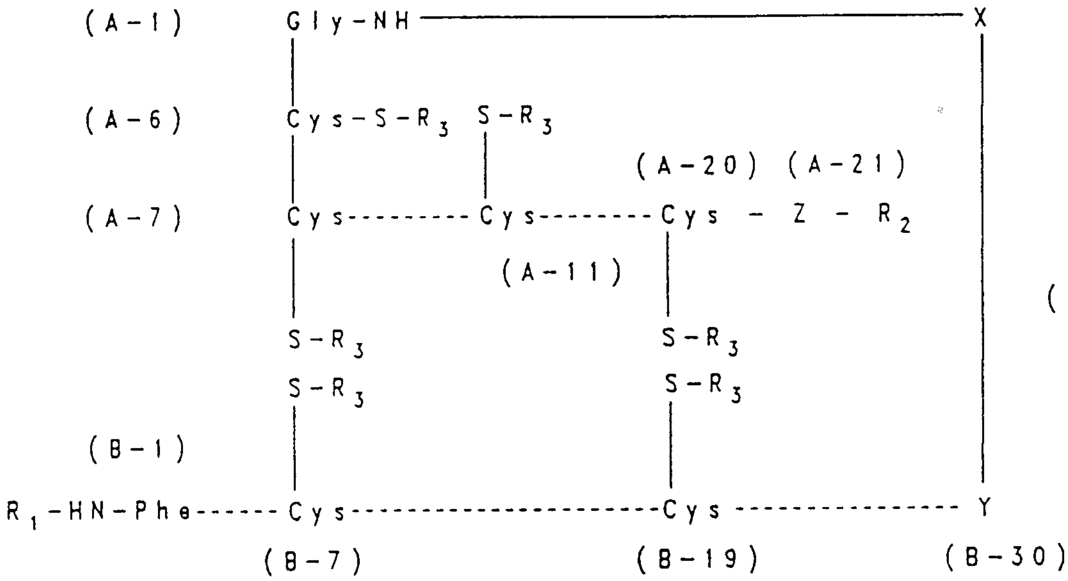

(II)

dans laquelle $R_1$, $R_2$, X, Y, Z, A1-A20 et B1-B29 ont les mêmes significations que dans la formule I et

$R_3$ = un groupe protecteur de S de Cys, de préférence le groupe $SO_3$,

avec un mercaptan en une quantité correspondant à un rapport SH (de mercaptan)/Cys-S-$R_3$ (dans la formule II) allant de 10 à 30, à un pH compris entre 9,5 et 11, en présence d'un système rédox organique ou d'au moins un composé organique qui, dans les conditions réactionnelles, forme un tel système rédox organique, choisi parmi l'acide ascorbique, l'acide déhydroascorbique, le pyrocatéchol, l'o-quinone, l'hydroquinone et la p-quinone, en utilisant le mercaptan et le(s) composé(s) formant le système rédox organique en un rapport de 1 équivalent-gramme de mercaptan à 0,1 - 10 modes de composé(s) formant le système rédox organique.

2. Procédé selon la revendication 1, caractérisé en ce que, comme mercaptan, on utilise le mercaptoéthanol et/ou la cystéine.

3. Procédé selon une ou plusieurs des revendications 1 et 2, caractérisé en ce que l'on opère en présence d'acide ascorbique et/ou d'acide déhydroascorbique.